# EUROPEAN PATENT APPLICATION

(11) **EP 4 394 034 A1**
(43) Date of publication of application: **03.07.2024**
(21) Application number: 22217314.8
(22) Date of filing: 30.12.2022
(51) Int. Cl.: C12N 9/10, C12N 15/52, C12N 15/82, C12N 15/62

(54) **ENGINEERED ACYLTRANSFERASES HAVING ALTERED SUBSTRATE SPECIFICITY**

(71) Applicant: kez.biosolutions GmbH, 14476 Potsdam (DE)
(72) Inventor: RITTNER, Alexander, Dr., 14476 Postdam-Golm (DE); DEGEN, Jan, 14476 Postdam-Golm (DE)
(74) Representative: Eisenführ Speiser

(57) **Abstract**

The present invention relates to the fields of fatty acid- and polyketide synthesis and protein engineering. In particular, it relates to the provision of engineered acyltransferases having altered substrate specificity as well as means and methods of using acyltransferases of the present invention for polyketide synthesis.

## Description

### Technical field

The present invention relates to the fields of fatty acid- and polyketide synthesis, and protein engineering. In particular, it relates to the provision of engineered acyltransferases having altered substrate specificity as well as means and methods of using acyltransferases of the present invention for polyketide synthesis.

### Background

Polyketides represent a versatile and important class of biomolecules. Particularly polyketide antibiotics, immunosuppressants, antiparasitics, as well as antifungal, cholesterol-lowering, and antitumoral agents represent important classes of biomolecules of outstanding therapeutic interest.

Despite having a biomolecular target, most polyketides are currently not used as therapeutics so that only a tiny fraction of their great potential is used for this particular substance class. In order to make them applicable for the human body, polyketides need to be tailored with certain functionalities. However, organic synthesis is very challenging and economically unfeasible for this particular substance class, due to their large carbon scaffolds with many functional groups and stereochemical properties. There is thus a great need in the pharmaceutical and biotechnological industry to provide systems allowing the synthesis of new and particularly custom-made polyketides with tailored functionalities in a reasonable time.

While polyketide synthase (PKS) systems bear a vast potential for synthetizing myriads of different biologically active compounds for drug design and other application, PKS and fatty acid synthase (FAS) enzymes are limited to substrates bound to Coenzyme A (CoA). CoA is composed of cysteamine (β-mercaptoethylamine), β-alanine, pantoic acid, organophosphate anhydride and a 3'-phosphoadenosine. The thiol group of the cysteamine can form a "high energy" thioester bond with carboxylic acids thereby forming "activated" acyl-CoA molecules.

CoA, however, is an abundant molecule in typical production strains. Consequently, having enzymes available with a decreased or even abolished specificity for CoA could be advantageous for the *in vivo* production of polyketides during metabolic engineering to shift the product yield towards a desired ketide or polyketide compound of interest using specifically designed enzymes as engineered molecular machines. In addition, using an artificial pathway (relying on acyl substrates bound to a different coenzyme moiety) for the biosynthesis of a target compound, avoids interference with the natural metabolism and a new functionality does no longer require identifying and establishing a suitable natural metabolic pathway to produce the substrate.

To our knowledge, there is no known acyltransferase that cannot bind CoA or would bind other coenzyme moieties. Re-engineering the coenzyme-binding properties, as in the present invention, is a highly challenging task and requires in-depth analysis, understanding and integration of the structural and functional properties as well as sequence-based information relating to the enzyme in context of the substrate binding. Especially, as the nucleotide binding pocket only forms during or after substrate binding and follows the "induced fit" concept. Hence, the binding pocket is only visible in crystal structures of acyltransferases in complex with their respective CoA ester substrates. This includes the identification of charged residues participating in electrostatic binding of the phosphate groups.

Crystal structures of some FAS enzymes and PKS systems have been solved (Smith and Tsai 2007, Rittner et al 2020). However, it has (to our knowledge) never been envisioned that the CoA binding pocket of an acyltransferase may be artificially redesigned so that it, ideally, does not use acyl-CoA substrates and accepts subunits bound to different coenzyme moieties, such as synthetic coenzyme moieties.

It was thus an object of the present invention to provide re-engineered acyltransferases, in particular those that are polyspecific in respect to the acyl moiety, for assisting in the production of polyketides and polyketide-precursors via biotechnological means to expand the currently available production methods. It was a particular object to expand and redefine the substrate recognition, binding and catalysis spectrum of acyltransferases to provide new acyltransferase enzymes, termed Selectases, capable of accepting a wide range of substrates, ideally while excluding CoA, to allow for targeted polyketide(-precursor) synthesis, preferably the synthesis of ketides or polyketide building blocks comprising positions within the polyketide molecule, where synthetic moieties can be inserted or added already during synthesis *in vivo* when produced by a host cell of interest, or by semi-synthetic processes after production of the (poly-)ketide scaffold.

### Definitions

An "acyltransferase" as used herein refers to an acyltransferase, including malonyl-acetyl-transferases (MATs), within and/or originating from a fatty acid synthase (FAS) or a polyketide synthase (PKS), wherein an acyltransferase of the present invention, also referred to as "Selectase" herein, is a reprogrammed acyltransferase originating from a FAS. An acyltransferase can selectively bind and trans-esterify different coenzyme-bound acyl molecules onto an Acyl carrier protein (ACP), for instance as part of fatty acid or polyketide synthesis. While natural acyltransferases, including FAS malonyl-acetyl-transferases (MATs) are specific for Coenzyme A (CoA)-bound acyl molecules, an acyltransferase of the present invention is an artificially reprogrammed acyltransferase that no longer accepts Acyl-CoA substrates and can instead bind and trans-esterify acyl groups bound to other coenzyme moieties, including synthetic coenzyme moieties. An acyltransferase of the present invention may be expressed as part of a FAS, or a sub-part thereof, as a separate molecule and/or as part of a fusion protein.

An acyltransferase "originating" from a fatty acid synthase refers to an acyltransferase for which the corresponding, wild type MAT is part of said fatty acid synthase in nature, wherein an acyltransferase of the present invention may be present as part of said fatty acid synthase or may be an isolated acyltransferase according to the present disclosure. For instance, for the reprogrammed acyltransferase according to SEQ ID NO: 57 the corresponding wild type MAT sequence is SEQ ID NO: 29, which is part of the fatty acid synthase according to SEQ ID NO: 1; hence the reprogrammed acyltransferase according to SEQ ID NO: 57 originates from the fatty acid sequence according to SEQ ID NO: 1. Various wild type MATs and the fatty acid synthases they are part of in nature, are disclosed as SEQ ID NO: 29 to 56 and SEQ ID NO: 1 to 28, respectively. The skilled person can easily determine, for instance through sequence alignments, the acyltransferase within a given FAS sequence. An acyltransferase may also be any fragment, part and/or (sub)domain of a sequence according to SEQ ID NO: 29 to 56 or a corresponding sequence of a different FAS, as long as it has acyltransferase activity.

An "isolated acyltransferase" as used herein, refers to an acyltransferase that is not present as part of a continuous polypeptide comprising the complete fatty acid synthase from which it originates, optionally not comprising more than the acyltransferase and optionally the linker domain (LD), or a part thereof, and/or the ketoacyl synthase (KS) of the fatty acid synthase from which it originates. An isolated acyltransferase may be present as a discrete enzyme, i.e. a polypeptide consisting of said acyltransferase. Preferably, a polypeptide comprising an isolated acyltransferase further comprises at least one protein, domain or part thereof to increase the stability, such as an LD, or a part thereof, wherein the LD may originate from the same FAS or may originate, in form of a fusion protein, from at least one different FAS and/or a polyketide synthase (PKS), or a different protein, domain or part thereof, for example an MBP-tag or a different tag. In certain embodiments, an isolated acyltransferase is present as part of a polypeptide comprising the acyltransferase, a KS as well as a LD connecting the acyltransferase and the KS, wherein the KS, the LD may originate from the same FAS or may originate, in form of a fusion protein, from at least one different FAS and/or a PKS. Generally, an isolated acyltransferase may be present as part of a fusion protein further comprising at least one polypeptide that does not originate from the same FAS or does not originate from any FAS, such one or more different enzyme parts and/or domains, including PKS functional domains, one or more tags, one or more binding domains, synthetic zippers and the like.

An "amino acid exchange" as used herein, refers to the presence of an amino acid at a given position of the primary amino acid sequence of a polypeptide that differs from the amino acid present at said position of the primary amino acid sequence of a reference polypeptide, such as a wild type polypeptide. Commonly, amino acid exchanges are achieved by mutating the respective codon of a nucleic acid sequence encoding the polypeptide into a codon encoding a different amino acid at said position. Means and methods to exchange an amino acid at a desired position, for instance but not limited to PCR-based cloning methods as disclosed herein, are well established in the field and available to the skilled person.

A "fatty acid synthase" or "FAS" as used herein, refers to an animal, including human, fatty acid synthase of type I, being a multi-functional protein, also called a multi-enzyme, capable of catalyzing all steps of fatty acid synthesis. In nature, wild type fatty acid synthases catalyze multiple rounds of Claisen condensation reactions with acetyl-CoA and malonyl-CoA recognized and transferred by an acyltransferase being a malonyl-acetyl-transferase (MAT) as substrates to synthesize fatty acids. The large multi-functional proteins comprise the functional domains of an acyltransferase transferring the acyl-substrates onto an acyl carrier protein (ACP), a ketoacyl synthase (KS) catalyzing the Claisen condensation reaction, a ketoacyl reductase (KR), hydroxyacyl dehydratase (DH) and enoyl reductase (ER) creating a fully saturated acyl backbone and a thioesterase (TE) releasing the synthesized fatty acid. (Smith and Tsai 2007).

A "fusion protein" as used herein refers to a polypeptide that is created by joining at least two different polypeptides, which may each be a complete protein existing in nature, a part thereof, or a fully synthetic polypeptide, to form one continuous artificial polypeptide. Commonly, a fusion protein is produced by fusing the nucleic acid sequences of the polypeptides to be joined. Means and methods to clone and express fusion proteins are well-established in the field and available to the skilled person. Fusion proteins of the present disclosure may for instance comprise one or more tags, such as affinity tags, including at least one HIS-tag, strep-tag, SNAP-tag, HA-tag and the like, or combinations thereof, e.g., an N-terminal HIS-tag and a C-terminally located strep-tag, or vice versa, epitope tags, fluorescence tags, solubilization tags, synthetic zippers or the like. A cleavable tag that can be removed after production may be used as well. Usually, tags will be located at the N- or C-terminus of a polypeptide not to disturb the function of the folded protein, but in certain cases, a tag may also be located within a polypeptide sequence (e.g., forming a discrete structural epitope not disturbing the fold and function of the polypeptide the tag is inserted in). Further, one or more linker sequences may be used, including a FAS and/or PKS LD, and/or other linkers (including synthetic linkers) or spacers or flexible linkers to connect the different entities of a fusion molecule with each other so that the different entities can exert there function appropriately (e.g., "GA" linkers or spacers of various length building alpha-helical structures may be used, one or more binding or docking domains, one or more dimerization domains, such as leucine zippers).

Whenever the present disclosure relates to the percentage of the identity of nucleic acid or amino acid sequences, this identity is determined by a comparison of a sequence of interest, the reference sequence (e.g., a SEQ ID NO as disclosed herein), to another sequence, the query sequence, over the entire length of the reference sequence. Identity is obtained by using the EMBOSS Water Pairwise Sequence Alignments (nucleotide) program or the EMBOSS Water Pairwise Sequence Alignments (protein) program (www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197).

When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

A "linker domain" or "LD" as used herein refers to both linker sequences upstream and downstream of an acyltransferase within a FAS or PKS that - together - link the acyltransferase to the KS, both sub-parts also being referred to as KS-AT (or KS-MAT) Linker and post-AT Linker. A linker domain as disclosed herein may comprise or consist of both sub-parts from the same FAS or PKS or a linker domain may be hybrid comprising or consisting of a KS-(M)AT Linker and a post-AT Linker, each stemming from a different FAS or PKS.

A "negatively charged" amino acid as used herein, refers to aspartate, glutamate, histidine or a non-natural amino acid or amino acid analog carrying a negative net charge at least at certain pH conditions. The terms "aspartate" and "glutamate" are used interchangeably with the terms "aspartic acid" and "glutamic acid", respectively, herein, irrespective of the protonation state.

Notably, histidine can be negatively or positively charged depending on the pH. Further, a neutral charge already at pH ~ 8.0 is possible. Therefore, depending on the pH and in view of the pl, histidine can be qualified as negatively charged residue, but also differently.

A "reference position" as used herein, refers to one of the conserved positions (i), (ii), (iii), (iv), (v), (vi), (vii) or (viii) as defined in Table 1. For an acyltransferase originating, for example, from a murine FAS according to SEQ ID NO: 1, the reference positions (i) to (viii) correspond to the positions T161, F184, R286, K186, K285, R300, A282, and G137 with the numbering based on the sequence of the acyltransferase domain itself (SEQ ID NO: 29), which corresponds to positions T648, F671, R773, K673, K772, R787, A769, and G624 with the numbering based on the sequence of the complete murine FAS (SEQ ID NO: 1). For an acyltransferase originating from a chicken FAS according to SEQ ID NO: 14, as a further example, the reference positions (i) to (viii) correspond to the positions T161, F184, R286, K186, R285, K300, A282, and G137 with the numbering based on the sequence of the acyltransferase domain itself (SEQ ID NO: 42), which corresponds to positions T647, F670, R772, K672, R771, K786, A769 and G623 with the numbering based on the sequence of the complete chicken FAS (SEQ ID NO: 14). When comparing different sequences of acyltransferases according to the present disclosure (e.g. SEQ ID NOs: 29 to 56) numbers of positions corresponding to the same reference position are frequently identical (when the numbering is based on the acyltransferase domain itself) but may also differ.

The reference positions disclosed herein are not limited to the sequences of SEQ ID NOs: 1 to 28 or SEQ ID NOs: 29 to 56. Sequence alignments, such as shown in **Table 1,** are well-established in the field and the skilled person can easily determine for any given FAS sequence, or the acyltransferase sequence therein, the positions of reference positions (i), (ii), (iii), (iv), (v), (vi), (vii) and/or (viii) within that sequence and further determine whether there is a threonine, serine or asparagine residue at reference position (i), a phenylalanine residue at reference position (ii), an arginine residue at reference position (iii), a lysine or arginine residue at reference position (iv), a lysine or arginine residue at reference position (v), a lysine or arginine residue at reference position (vi), an alanine residue at reference position (vii), and/or a glycine residue at reference position (viii).

Whenever the present disclosure refers to an "analogous" residue in the context to SEQ ID NO: 29, it refers to the residue present in a different sequence at the position that corresponds to the same reference position. For example, T161 of SEQ ID NO: 14 is an analogous threonine residue to T161 of SEQ ID NO: 29 and R285 of SEQ ID NO 14 is an analogous arginine residue to K285 of SEQ ID NO: 29. While the numbering based on SEQ ID NO: 14 is identical to the numbering based on SEQ ID NO: 29, an analogous residue may not necessarily have the identical number as the respective residue in SEQ ID NO: 29, depending on the individual sequence.

The terms "polypeptide" and "protein" are used interchangeably herein. Naturally, protein functionality, including enzymatic activity, is dependent on further factors, such as temperature, pH, salts, and the like and may require further proteins and/or co-factors as it is known to the skilled person. Polypeptides disclosed herein may start with an additional methionine residue resulting from translation of the start codon, or alternatively with a different additional residue, such as valine or leucine resulting from translation of an alternative start codon, likewise polypeptides disclosed herein as beginning with a methionine residue may also be present without said methionine e.g. if used in context of an N-terminal fusion to a tag or other polypeptide.

### Brief description of the Drawings

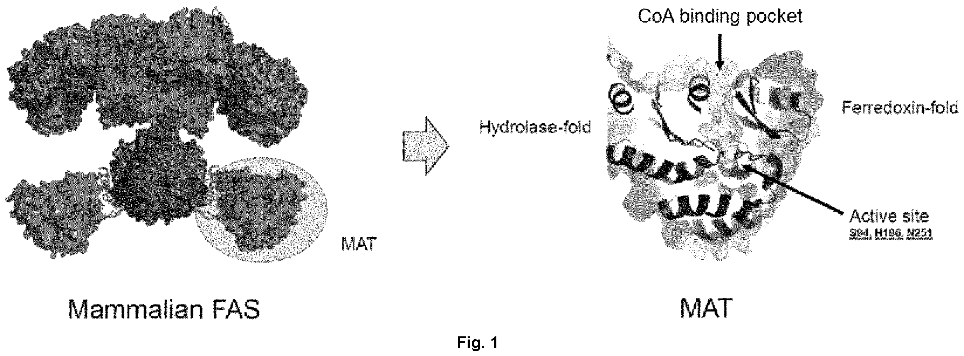

**Figure 1 (Fig. 1****)** shows a cartoon of the structure of a mammalian FAS homodimer with one of the Malonyl-Acetyl-Transferases (MAT) highlighted as well as a corresponding cartoon showing the position of the CoA binding channel within a MAT as a cartoon. The numbering of the residues of the active site (S94, H196 and N251) is based on SEQ ID NO: 29.

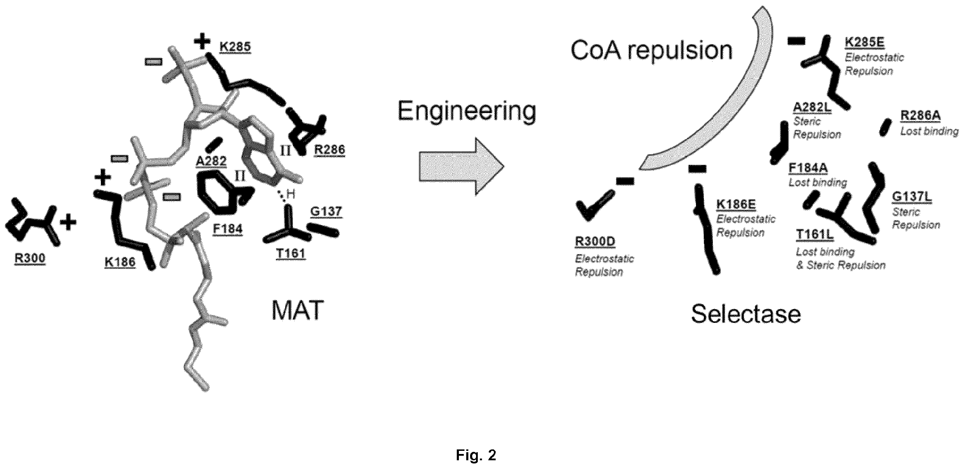

**Figure 2 (Fig. 2****)** shows the residues of a murine MAT (SEQ ID NO: 29) identified in the structural as well as sequence-based analysis for targeted reprogramming of the coenzyme A binding. Structural sketches of the eight different identified residues are shown in black with the positively charged residues (K186, K285 and R300) being marked with a black "+". A structural sketch of the CoA is shown in gray, with the negatively charged phosphate groups being marked with a gray "-". Pi-stacking between the CoA-Adenine and the residues F184 and R286 are indicated as "π". H-bonding between residue T161 and the CoA-Adenine is indicated with a dotted line and an "H".

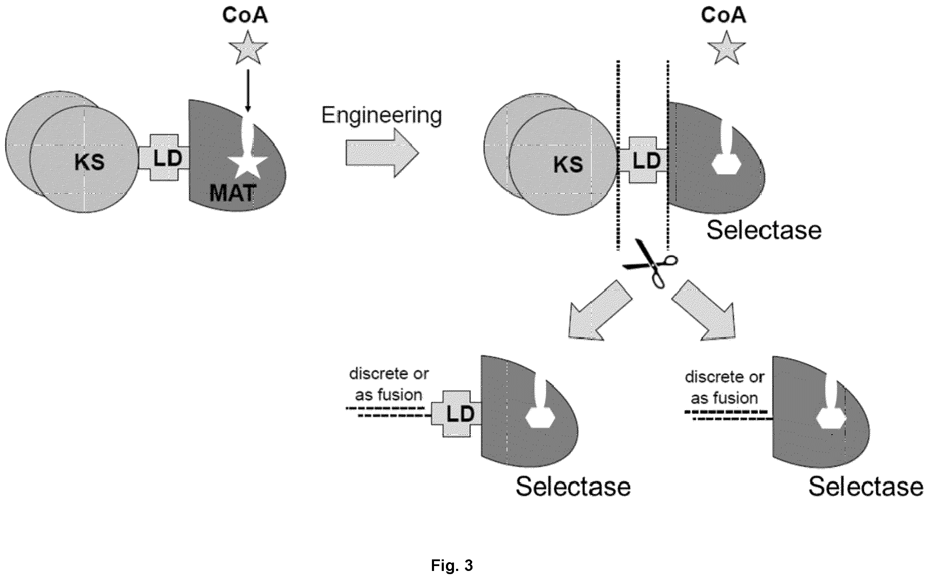

**Figure 3 (Fig. 3****)** shows an exemplary scheme of engineering a Malonyl-Acetyl-Transferase (MAT) into a reprogrammed acyltransferase having altered substrate specificity. In the top left is a sketch of a KS-LD-MAT polypeptide capable of binding CoA. In the top right is a sketch of an engineered KS-LD-acyltransferase according to the present invention. On the bottom right are examples of using a reprogrammed acyltransferase of the present invention.

### Summary of the inventions

In a first aspect, there is provided an acyltransferase having altered substrate specificity, wherein the acyltransferase comprises one or more amino acid exchange(s) at reference position(s) (i) T161 according to SEQ ID NO: 29, or an analogous threonine, serine or asparagine residue at said reference position as defined in Table 1, wherein the amino acid is exchanged to alanine, glycine, valine, leucine, isoleucine, tyrosine, phenylalanine or tryptophan; and/or (ii) F184 according to SEQ ID NO: 29, or an analogous phenylalanine residue at said reference position as defined in Table 1, wherein the amino acid is exchanged to alanine, glycine, valine, leucine, isoleucine or methionine; and/or (iii) R286 according to SEQ ID NO: 29, or an analogous arginine residue at said reference position as defined in Table 1, wherein the amino acid is exchanged to alanine, glycine, valine, leucine, isoleucine or methionine; and/or (iv) K186 according to SEQ ID NO: 29, or an analogous lysine or arginine residue at said reference position as defined in Table 1, wherein the amino acid is exchanged to a negatively charged amino acid or to glycine, alanine or valine; and/or (v) K285 according to SEQ ID NO: 29, or an analogous lysine or arginine residue at said reference position as defined in Table 1, wherein the amino acid is exchanged to a negatively charged amino acid; and/or (vi) R300 according to SEQ ID NO: 29, or an analogous lysine or arginine residue at said reference position as defined in Table 1, wherein the amino acid is exchanged to a negatively charged amino acid, and/or (vii) A282 according to SEQ ID NO: 29, or an analogous alanine residue at said reference position as defined in Table 1, wherein the amino acid is exchanged to valine, leucine, isoleucine, phenylalanine or tyrosine; and/or (viii) G137 according to SEQ ID NO: 29, or an analogous glycine residue at said reference position as defined in Table 1, wherein the amino acid is exchanged to valine, leucine, isoleucine, phenylalanine or tyrosine.

In some embodiments of the first aspect, an amino acid exchange at reference position (i) is an exchange to alanine, glycine, or valine, preferably to alanine; and/or an amino acid exchange at reference position (ii) is an exchange to alanine, glycine, or valine, preferably to alanine; and/or an amino acid exchange at reference position (iii) is an exchange to alanine, glycine, or valine, preferably to alanine; and/or an amino acid exchange at reference position (iv) is an exchange to a negatively charged amino acid, preferably to glutamate or aspartate; and/or an amino acid exchange at reference position (v) is an exchange to glutamate or aspartate; and/or an amino acid exchange at reference position (vi) is an exchange to glutamate or aspartate; and/or an amino acid exchange at reference position (vii) is an exchange to leucine, isoleucine, phenylalanine or tyrosine; and/or an amino acid exchange at reference position (viii) is an exchange to leucine, isoleucine, phenylalanine or tyrosine.

In some embodiments of the first aspect, an amino acid exchange at reference position (i) is an exchange to leucine, isoleucine, tyrosine, phenylalanine or tryptophan; and/or an amino acid exchange at reference position (ii) is an exchange to leucine, isoleucine or methionine; and/or an amino acid exchange at reference position (iii) is an exchange to leucine, isoleucine or methionine; and/or an amino acid exchange at reference position (iv) is an exchange to alanine, glycine, or valine; and/or an amino acid exchange at reference position (vii) is an exchange to valine; and/or an amino acid exchange at reference position (viii) is an exchange to valine.

In preferred embodiments of the first aspect, the acyltransferase is an isolated acyltransferase originating from a fatty acid synthase.

In some embodiments of the first aspect, the acyltransferase comprises amino acid exchanges as defined above at reference positions (iv) and (i), or (iv) and (ii), or (iv) and (iii), or (iv) and (v), or (iv) and (vi), or (iv) and (vii), or (iv) and (viii), or (iv) and two or more of reference positions of (i), (ii), (iii), (v), (vi), (vii) and/or (viii), optionally wherein the amino acid exchange at reference position (iv) is an amino acid exchange to glutamate or aspartate.

In some embodiments of the first aspect, the acyltransferase comprises amino acid exchanges as defined above at reference positions (i) and (ii), or (i) and (iii), or (i) and (iv), or (i) and (v), or (i) and (vi), or (i) and (vii), or (i) and (viii), or (i) and two or more of reference positions of (ii), (iii), (iv), (v), (vi), (vii) and/or (viii), optionally wherein the amino acid exchange at reference position (i) is an amino acid exchange to alanine.

In some embodiments of the first aspect, the acyltransferase comprises amino acid exchanges as defined above at reference positions: (i) and (ii) and (iii) and optionally (iv), preferably wherein the amino acids at said reference positions are exchanged to alanine; or (ii) and (iii) and optionally (i), preferably wherein the amino acids at said reference positions are exchanged to alanine; or (iv) and (vi), preferably wherein the amino acid exchanges at said reference positions are amino acid exchanges to glutamate at reference positions (iv) and (vi), or amino acid exchanges to aspartate at reference positions (iv) and (vi), or an amino acid exchange to glutamate at reference position (iv) and an amino acid exchange to aspartate at reference position (vi), or an amino acid exchange to aspartate at reference position (iv) and an amino acid exchange to glutamate at reference position (vi), optionally further comprising amino acid exchanges as defined above at reference positions (i) and (ii) and (iii), preferably amino acid exchanges to alanine at reference positions (i) and (ii) and (iii).

In some embodiments of the first aspect, the acyltransferase originates from a fatty acid synthase of an organism selected from vertebrata, including aves and mammalia, including artiodactyla, perissodactyla, carnivora, primates and rodentia, preferably wherein the acyltransferase originates from a fatty acid synthase comprising or consisting of a wild type amino acid sequence selected from any one of SEQ ID NO: 1 to 28, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

In some embodiments of the first aspect, the acyltransferase comprises or consists of an amino acid sequence of any of SEQ ID NO: 57 to 82, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

In a second aspect, there is provided a fusion protein comprising at least one acyltransferase of any one of the embodiments of the first aspect, preferably wherein the fusion protein further comprises at least one linker domain and optionally at least one ketoacyl synthase domain.

In a third aspect, there is provided a nucleic acid encoding the acyltransferase of the first aspect, or the fusion protein of the third aspect.

In a fourth aspect, there is provided an expression construct or vector comprising at least one nucleic acid of the third aspect.

In a fifth aspect, there is provided a cell comprising at least one acyltransferase of the first aspect; and/or at least one fusion protein of the second aspect; and/or at least one nucleic acid of the third aspect; and/or at least one expression construct or vector of the fourth aspect.

In a sixth aspect, there is provided a kit comprising at least one acyltransferase of the first aspect; and/or at least one fusion protein of the second aspect; and/or at least one nucleic acid of the third aspect; and/or at least one expression construct or vector of the fourth aspect; and/or at least one cell of the sixth aspect; optionally further comprising at least one container; and/or a set of reagents including buffer, medium and/or substrate(s); and/or means of transfection. A kit will usually comprise all agents, buffers, co-factors etc. necessary to guarantee the functionality of the at least one acyltransferase in an assay of interest.

In a seventh aspect, there is provided a use of at least one acyltransferase as defined in the first aspect; and/or at least one fusion protein as defined in the second aspect; and/or at least one nucleic acid as defined in the third aspect; and/or at least one expression construct or vector as defined in the fourth aspect; and/or at least one cell as defined in the fifth aspect; and/or at least one kit as defined in the sixth aspect; for polyketide synthesis, fatty acid synthesis and/or non-ribosomal peptide synthesis, optionally wherein it is a use for reacting at least two different substrates.

### Detailed description

The present invention will now be described in detail based on the detailed description, including non-limiting Examples, the attached drawings and the sequences as provided with the sequence listing.

In a first aspect, there is provided an acyltransferase having altered substrate specificity, wherein the acyltransferase comprises one or more amino acid exchange(s) at reference position(s) (i) T161 according to SEQ ID NO: 29, or an analogous threonine, serine or asparagine residue at said reference position as defined in Table 1, wherein the amino acid is exchanged to alanine, glycine, valine, leucine, isoleucine, tyrosine, phenylalanine or tryptophan; and/or (ii) F184 according to SEQ ID NO: 29, or an analogous phenylalanine residue at said reference position as defined in Table 1, wherein the amino acid is exchanged to alanine, glycine, valine, leucine, isoleucine or methionine; and/or (iii) R286 according to SEQ ID NO: 29, or an analogous arginine residue at said reference position as defined in Table 1, wherein the amino acid is exchanged to alanine, glycine, valine, leucine, isoleucine or methionine; and/or (iv) K186 according to SEQ ID NO: 29, or an analogous lysine or arginine residue at said reference position as defined in Table 1, wherein the amino acid is exchanged to a negatively charged amino acid or to glycine, alanine or valine; and/or (v) K285 according to SEQ ID NO: 29, or an analogous lysine or arginine residue at said reference position as defined in Table 1, wherein the amino acid is exchanged to a negatively charged amino acid; and/or (vi) R300 according to SEQ ID NO: 29, or an analogous lysine or arginine residue at said reference position as defined in Table 1, wherein the amino acid is exchanged to a negatively charged amino acid, and/or (vii) A282 according to SEQ ID NO: 29, or an analogous alanine residue at said reference position as defined in Table 1, wherein the amino acid is exchanged to valine, leucine, isoleucine, phenylalanine or tyrosine; and/or (viii) G137 according to SEQ ID NO: 29, or an analogous glycine residue at said reference position as defined in Table 1, wherein the amino acid is exchanged to valine, leucine, isoleucine, phenylalanine or tyrosine.

Wild type (i.e. without comprising the one or more amino acid exchanges of the present invention) acyltransferases from fatty acid synthases can transfer different acyl-groups from acyl-coenzyme A (CoA) substrates to an acyl carrier protein. While they are polyspecific for multiple different acyl groups, they are specific for CoA-bound substrates. The two different types of specificity, the CoA specificity and the acyl polyspecificity, are conferred by different residues of the enzyme. The residues at the reference positions are crucial for binding to the CoA moiety of the substrate and the amino acid exchanges of the present invention can be used to alter these binding properties and engineer acyltransferases that can bind to substrates covalently bound to moieties other than CoA, including synthetic coenzyme moieties. Acyltransferases having altered substrate specificity according to the present invention are acyltransferases having a reduced ability, preferably essentially no ability, to transfer CoA-bound substrates, while the acyl polyspecificity may be unaltered.

The residues at reference positions (i), (ii) and (iii) are crucial residues for the binding pocket of the respective coenzyme (see **Fig. 2**) and interact with the nucleobase portion of the CoA moiety through hydrogen bonding (reference position (i)) or pi-stacking (reference positions (ii) and (iii)). One or more amino acid exchange(s) at reference positions (i), (ii) and/or (iii) to alanine, glycine and/or valine may be used to prevent the H-bonding and/or Pi-stacking. An amino acid exchange at reference position (i) to leucine, isoleucine, tyrosine, phenylalanine or tryptophan may be used to sterically manipulate and reprogram the binding to coenzyme moieties, wherein an exchange to tyrosine, phenylalanine or tryptophan further may be used to stabilize the protein through pi-stacking. The amino acid exchange(s) at reference position (ii) and/or (iii) to leucine, isoleucine or methionine may be used to sterically manipulate the binding to coenzyme moieties.

The lysine or arginine residues at reference positions (iv), (v) and (vi) interact electrostatically with the negatively charged phosphate groups of CoA. Exchanging one or more residues at reference positions (iv), (v) and/or (vi) to one or more negatively charged residue(s) may be used to engineer acyltransferases that, instead, bind to one or more positively charged chemical groups of coenzyme moieties other than CoA, including synthetic coenzyme moieties.

An amino acid exchange at reference position (iv) to alanine, glycine and/or valine may be used to artificially eliminate the electrostatic interaction at this reference position.

The alanine residue at reference position (vii) and the glycine residue at reference position (viii) form structural parts of the coenzyme binding pocket. Exchanging the residues at one and/or both of these positions may be used to manipulate the structural shape of the coenzyme binding pocket through modification of the steric arrangement.

Preferred positions for engineering a Selectase of the present invention are reference positions (ii), (iii), (v) and/or (vii), even more preferred is reference position (vi) and most preferred are reference positions (i) and/or (iv).

In some embodiments of the first aspect, an amino acid exchange at reference position (i) is an exchange to alanine, glycine, or valine, preferably to alanine; and/or an amino acid exchange at reference position (ii) is an exchange to alanine, glycine, or valine, preferably to alanine; and/or an amino acid exchange at reference position (iii) is an exchange to alanine, glycine, or valine, preferably to alanine; and/or an amino acid exchange at reference position (iv) is an exchange to a negatively charged amino acid, preferably to glutamate or aspartate; and/or an amino acid exchange at reference position (v) is an exchange to glutamate or aspartate; and/or an amino acid exchange at reference position (vi) is an exchange to glutamate or aspartate; and/or an amino acid exchange at reference position (vii) is an exchange to leucine, isoleucine, phenylalanine or tyrosine; and/or an amino acid exchange at reference position (viii) is an exchange to leucine, isoleucine, phenylalanine or tyrosine.

In some embodiments of the first aspect, an amino acid exchange at reference position (i) is an exchange to leucine, isoleucine, tyrosine, phenylalanine or tryptophan; and/or an amino acid exchange at reference position (ii) is an exchange to leucine, isoleucine or methionine; and/or an amino acid exchange at reference position (iii) is an exchange to leucine, isoleucine or methionine; and/or an amino acid exchange at reference position (iv) is an exchange to alanine, glycine, or valine; and/or an amino acid exchange at reference position (vii) is an exchange to valine; and/or an amino acid exchange at reference position (viii) is an exchange to valine.

In preferred embodiments of the first aspect, the acyltransferase is an isolated acyltransferase originating from a fatty acid synthase.

In one embodiment, the isolated acyltransferase is a polypeptide or part of a polypeptide comprising or consisting of the acyltransferase, without comprising further parts and/or domains, including a LD, of the FAS from which it originates. The isolated acyltransferase may be part of a fusion protein according to the second aspect.

In another embodiment, the isolated acyltransferase is part of a polypeptide comprising or consisting of the acyltransferase and a LD, or a part thereof, without comprising further parts and/or domains of the FAS from which it originates. The isolated acyltransferase may be part of a fusion protein according to the second aspect.

In another embodiment, the isolated acyltransferase is part of a polypeptide comprising or consisting of the portion of a FAS spanning the ketoacyl synthase (KS), the acyltransferase and the LD connecting the KS and the acyltransferase, without comprising further parts and/or domains of the FAS from which it originates. Such a KS-LD-acyltransferase may be part of a fusion protein according to the second aspect.

In some embodiments of the first aspect, the acyltransferase comprises amino acid exchanges as defined above at reference positions (iv) and (i), or (iv) and (ii), or (iv) and (iii), or (iv) and (v), or (iv) and (vi), or (iv) and (vii), or (iv) and (viii), or (iv) and two or more of reference positions of (i), (ii), (iii), (v), (vi), (vii) and/or (viii), optionally wherein the amino acid exchange at reference position (iv) is an amino acid exchange to glutamate or aspartate.

In some embodiments of the first aspect, the acyltransferase comprises amino acid exchanges as defined above at reference positions (i) and (ii), or (i) and (iii), or (i) and (iv), or (i) and (v), or (i) and (vi), or (i) and (vii), or (i) and (viii), or (i) and two or more of reference positions of (ii), (iii), (iv), (v), (vi), (vii) and/or (viii), optionally wherein the amino acid exchange at reference position (i) is an amino acid exchange to alanine.

In some embodiments of the first aspect, the acyltransferase comprises amino acid exchanges as defined above at reference positions: (i) and (ii) and (iii) and optionally (iv), preferably wherein the amino acids at said reference positions are exchanged to alanine; or (ii) and (iii) and optionally (i), preferably wherein the amino acids at said reference positions are exchanged to alanine; or (iv) and (vi), preferably wherein the amino acid exchanges at said reference positions are amino acid exchanges to glutamate at reference positions (iv) and (vi), or amino acid exchanges to aspartate at reference positions (iv) and (vi), or an amino acid exchange to glutamate at reference position (iv) and an amino acid exchange to aspartate at reference position (vi), or an amino acid exchange to aspartate at reference position (iv) and an amino acid exchange to glutamate at reference position (vi), optionally further comprising amino acid exchanges as defined above at reference positions (i) and (ii) and (iii), preferably amino acid exchanges to alanine at reference positions (i) and (ii) and (iii).

In some embodiments of the first aspect, the acyltransferase originates from a fatty acid synthase of an organism selected from vertebrata, including aves and mammalia, including artiodactyla, perissodactyla, carnivora, primates and rodentia, preferably wherein the acyltransferase originates from a fatty acid synthase comprising or consisting of a wild type amino acid sequence selected from any one of SEQ ID NO: 1 to 28, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

In some embodiments of the first aspect, the acyltransferase comprises or consists of an amino acid sequence of any of SEQ ID NO: 57 to 82, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

In a second aspect, there is provided a fusion protein comprising at least one acyltransferase of any one of the embodiments of the first aspect, preferably wherein the fusion protein further comprises at least one linker domain and optionally at least one ketoacyl synthase domain.

In some embodiments, the fusion protein may comprise a tag for purification, for example but not limited to at least one His-tag, step-tag, FLAG-tag, myc-tag, MBP-tag, GST-tag, or a combination thereof.

The acyltransferase of the present invention of preferably linked to other functional PKS or FAS parts and/or domains, in particular a ketoacyl synthase domain, via a FAS and/or PKS LD. Of course, the acyltransferase may also be linked to other functional parts and/or domains by conventional polypeptide linkers known in the art, such as glycine linkers, GS linkers, PT-linkers or other commonly used linkers.

In some embodiments, the fusion protein may be a polyketide synthase (PKS), or a part thereof, in which at least one acyltransferase has been replaced by an acyltransferase of the present invention, wherein the PKS portion of the fusion protein may comprise further engineered modifications.

Type I PKSs are large multifunctional proteins that have a modular enzymatic structure, in which one module comprises a set of functional enzymatic domains involved in the incorporation of an α-carboxyacyl extender unit into a growing polyketide chain. A type I PKS module comprises at least three different functional domains: a ketoacyl synthase (KS), which is responsible for catalyzing the Claisen condensation between an extender unit and the growing polyketide chain; an acyltransferase, which selectively binds an extender unit and trans-esterifies the extender onto an acyl carrier protein (ACP); the ACP serves as the acceptor for the extender unit and the growing polyketide chain. A type I PKS may further comprise additional domains, such as a ketoacyl reductase (KR), which reduces the β-ketone to a hydroxyl group, a dehydratase (DH) which removes the hydroxyl group and creates a double bond between the α and β carbon, an enoyl reductase (ER) which reduces the double bond generated by the DH domain, and a thioesterase (TE) terminating the synthesis process. A type I PKS may be an iterative PKS, catalyzing multiple elongation cycles repeatedly using the same functional domains, or it may be an assembly-line PKS comprising different modules, each of which catalyzes one elongation step (Hertweck 2009, Cogan *et al.* 2021).

Type II PKS systems comprise discrete proteins, each individually comprising enzymatic activities for polyketide synthesis.

Usually, the starter unit or the extender unit is a CoA-bound acyl group, such as acetyl-CoA, malonyl-CoA, methylmalonyl-CoA or propionyl-CoA, which are selectively recognized and transferred to the ACP by the acyltransferase. By exchanging the acyltransferase to an engineered acyltransferase of the present invention, a PKS system may be reprogrammed to incorporate extender units bound to other coenzyme moieties, including synthetic coenzyme moieties. The acyltransferase of a PKS may be replaced by an acyltransferase of the present invention through regular cloning strategies available to the skilled person.

In modular, assembly-line PKS systems, the acyltransferase of one, or two, or more, or all modules may be replaced by a reprogrammed acyltransferase of the present invention, optionally including the adjacent LD, or a part thereof, and/or KS, to produce a reprogrammed PKS system capable of using extender units bound by coenzyme moieties other than CoA.

An acyltransferase or fusion protein according to the various embodiments of the first or second aspect, may be purified using protein purification methods disclosed herein or any other conventional protein purification methods.

In a third aspect, there is provided a nucleic acid encoding the acyltransferase of the first aspect, or the fusion protein of the third aspect.

The nucleic acid may be double or single stranded DNA or RNA, including mRNA. A nucleic acid that is not part of an expression construct or vector may comprise other sequences and/or modification for improving the stability.

In a fourth aspect, there is provided an expression construct or vector comprising at least one nucleic acid of the third aspect.

The nucleic acid of the third aspect and the expression construct or vector of the fourth aspect may further comprise sequences for expression in a desired host cell, such as a promoter, including inducible promoters, and/or a transcription terminator operably linked to the nucleic acid encoding the acyltransferase or the fusion protein, a selectable marker, a sequence for replication in a desired host cell, and/or further regulatory sequences, including sequences regulatory for transcription, RNA processing and/or stability, and/or translation. The nucleic acid or expression construct or vector may comprise a cDNA sequence, devoid of introns, encoding the acyltransferase and/or fusion protein of the present invention, e.g. for expression in prokaryotic cells. Recombinant protein expression, as well as suitable vectors and expression systems, promoters and further regulatory sequences are well-established for a multitude of cell types. The skilled person is wellaware of the particulars and requirements for recombinant protein expression in different cell types and can easily determine suitable components to choose for expression in the desired host cell. Vector backbones for an expression construct or vector may for example be pET22b, pET300, pET301, pET28b or pGEX-6P-1 vector systems, but are not limited thereto.

In certain embodiments of the third or fourth aspect, the nucleic acid or expression construct or vector is codon optimized, wherein the codon optimization may be adapted according to the desired host cell. Codon optimizations for different cell types are well-established in the art and tools for codon optimization are readily available to the skilled person.

The nucleic acid of the third aspect and/or the expression construct or vector of the fourth aspect may be suitable for stable replication and maintenance in the desired host cell. The nucleic acid and/or expression construct or vector may further be suitable for integration into the host genome.

In a fifth aspect, there is provided a cell comprising at least one acyltransferase of the first aspect; and/or at least one fusion protein of the second aspect; and/or at least one nucleic acid of the third aspect; and/or at least one expression construct or vector of the fourth aspect.

In one embodiment, at least one nucleic acid encoding at least one acyltransferase of the first aspect and/or at least one fusion protein of the second aspect may be stably integrated into the genome, including the chromosomal/nuclear genome and/or the plastid genome, of the host cell, e.g. by any conventional genome editing strategy and/or any conventional vector suitable for genome integration. In certain embodiments, the at least one nucleic acid of the present invention may be integrated into the genome by replacing an endogenous nucleic acid encoding an acyltransferase.

In another embodiment, an endogenous gene of a host cell encoding an acyltransferase is mutated to encode an acyltransferase of the present invention, wherein the mutation may be performed by any mutation strategy, preferably a SDN-1 (site directed nuclease 1) mutation strategy.

In another embodiment, at least one nucleic acid of the third aspect and/or at least one expression construct or vector of the fourth aspect is present extrachromosomally in the host cell.

An acyltransferase of the present invention may be expressed in any cellular expression system, established systems include prokaryotic cells, including *Escherichia coli* strains, such as but not restricted to various different well-established BL21 or BL21(DE3) expression strains as well as derivatives thereof, including for example Rosetta, BAP1 or NiCo21 (DE3) strains, other *E*. *coli* strains, such as *E*. *coli* K12 strain JM83 or other prokaryotic cells such as *Bacillus subtilis* expression systems, eukaryotic expression systems, for example including *Saccharomyces cerevisiae* cells, *Komagataella phaffii* (formerly called *Pichia pastoris)* cells, *Aspergillus niger* cells, or immortalized cell lines, such as insect cell expression systems, for example Sf-9 or Sf-21 cells, or mammalian cell lines, such Chinese hamster Ovary (CHO) cells, HEK 293 cells and the like.

In one embodiment, at least one endogenous acyltransferase of the cell is knocked out, knocked down or otherwise inactivated and complemented with at least one acyltransferase and/or fusion protein of the present invention; and/or at least one nucleic acid, expression construct and/or vector encoding the same.

In preferred embodiments, the cell comprises a PKS system in which at least one acyltransferase has been replaced with an acyltransferase of the present invention. In certain embodiments, the cell comprises a modular type I PKS system in which the acyltransferase of one, or two, or more, or all PKS modules has been replaced by a reprogrammed acyltransferase of the present invention, optionally including the adjacent LD, or a part thereof, and/or KS, of the present invention.

In a sixth aspect, there is provided a kit comprising at least one acyltransferase of the first aspect; and/or at least one fusion protein of the second aspect; and/or at least one nucleic acid of the third aspect; and/or at least one expression construct or vector of the fourth aspect; and/or at least one cell of the sixth aspect; optionally further comprising at least one container; and/or a set of reagents including buffer, medium and/or substrate(s); and/or means of transfection. A kit will usually comprise all agents, buffers, co-factors etc. necessary to guarantee the functionality of the at least one acyltransferase in an assay of interest.

In a seventh aspect, there is provided a use of at least one acyltransferase as defined in the first aspect; and/or at least one fusion protein as defined in the second aspect; and/or at least one nucleic acid as defined in the third aspect; and/or at least one expression construct or vector as defined in the fourth aspect; and/or at least one cell as defined in the fifth aspect; and/or at least one kit as defined in the sixth aspect; for polyketide synthesis, fatty acid synthesis and/or non-ribosomal peptide synthesis, optionally wherein it is a use for reacting at least two different substrates.

As a Selectase according to the first aspect comprises specifically reprogrammed coenzyme-binding properties without the necessity to alter amino acid residues involved in the acyl moiety binding, the acyltransferase of the present invention can retain the natural polyspecificity regarding the acyl moiety. Retaining the acyl polyspecificity can be of great importance, e.g. for using one acyltransferase for different acyl-moieties as substrates, in particular when using at least two different substrates for polyketide synthesis, fatty acid synthesis and/or non-ribosomal peptide synthesis.

"Polyketide synthesis" as used herein includes synthesis of diketides and oligoketides.

In certain embodiments, the polyketide synthesis, fatty acid synthesis and/or nonribosomal peptide synthesis may be a synthesis of polyketide, and/or fatty acid and/or protein hybrids with metabolites of other biosynthetic synthesis pathways.

In one embodiment, synthesis of the seventh aspect is *in vivo* synthesis in at least one cell according to the fifth aspect, further using at least one suitable medium and at least one reaction substrate comprising a coenzyme moiety other than CoA, the at least one cell thus representing an engineered production strain.

In another embodiment, synthesis of the seventh aspect is *in vitro* synthesis using at least one purified or partially purified acyltransferase of the first aspect and/or at least one fusion protein of the second aspect, at least one buffer, at least one reaction substrate comprising a coenzyme moiety other than CoA and optionally further suitable reaction components.

In another embodiment, synthesis of the seventh aspect is *in vitro* synthesis using lysate of at least one cell according to the fifth aspect, at least one reaction substrate comprising a coenzyme moiety other than CoA, at least one buffer and optionally further suitable reaction components.

In another embodiment, synthesis of the seventh aspect is *in vitro* synthesis using at least one *in vitro* transcription/translation system expressing at least one nucleic acid of the third aspect and/or at least one expression construct or vector of the fourth aspect, at least one reaction substrate comprising a coenzyme moiety other than CoA and optionally at least one buffer and/or further suitable reaction components.

### Examples

### Example 1: In silico design for targeted acyltransferase reprogramming

Due to their modular nature and their innate variability in acyl backbone reduction, PKS systems are an enticing enzymatic platform to be exploited for synthesizing a plethora of different useful biomolecules, in particular for drug design. A crucial aspect limiting a versatile and effective biotechnological use of PKS is the nature of naturally occurring acyltransferases, which have a relatively high specificity when it comes to the extender subunits (Smith and Tsai 2007). The MAT domains of metazoan FAS enzymes were shown to allow an efficient transfer of different acyl substrates (Rittner *et al.* 2018).

However, while a FAS MAT might therefore offer a certain versatility regarding the acyl group of the substrate, both FAS MATs and PKS ATs suffer from a crucial shortcoming: they are fully dependent on CoA-bound substrates. The dependency on Acyl-CoA substrates strongly limited the possibility for artificial biomolecule production using an engineered PKS system for *in vivo* synthesis. CoA-bound molecules, in particular Acetyl-CoA and Malonyl-CoA, are highly abundant and unavoidable in any *in vivo* setting, thereby interfering with any possible biosynthesis based on CoA-bound substrates. While the MAT crystal structure is solved (Rittner *et al.* 2020), it has not been envisioned that the crucial activating component of CoA may be replaced, let alone how an acyltransferase may be artificially reprogrammed so that it does not use Acyl-CoA substrates and, instead, specifically accepts subunits bound to different coenzyme moieties, such as synthetic coenzyme moieties.

Based on the crystal structure and the position of the CoA binding pocket therein (see Fig. 1), it was speculated that target residues that are highly attractive to reprogramming a natural acyltransferase could be identified. However, mutation experiments showed that amino acid exchanges at certain residues do not allow any functional protein expression. Therefore, an iterative testing strategy and protein engineering techniques combined with in silico searches were applied to define acyltransferase mutants that can be manipulated in a way to efficiently block CoA binding and -at the same time - that allow binding of different coenzyme moieties, while still allowing correct overall folding of the protein. Moreover, to offer a high versatility including the choice of acyltransferase protein itself, alignments of FAS amino acid sequences from various genera were performed in addition to the structural analysis. An alignment of 28 different example sequences is shown in **Table 1**. After extensive analysis, eight different target residues have been identified (see **Fig. 2**) in those sequences showing a certain degree of identity. To this end, various hot spots for mutational activities were defined that were tested in the following as further detailed below.

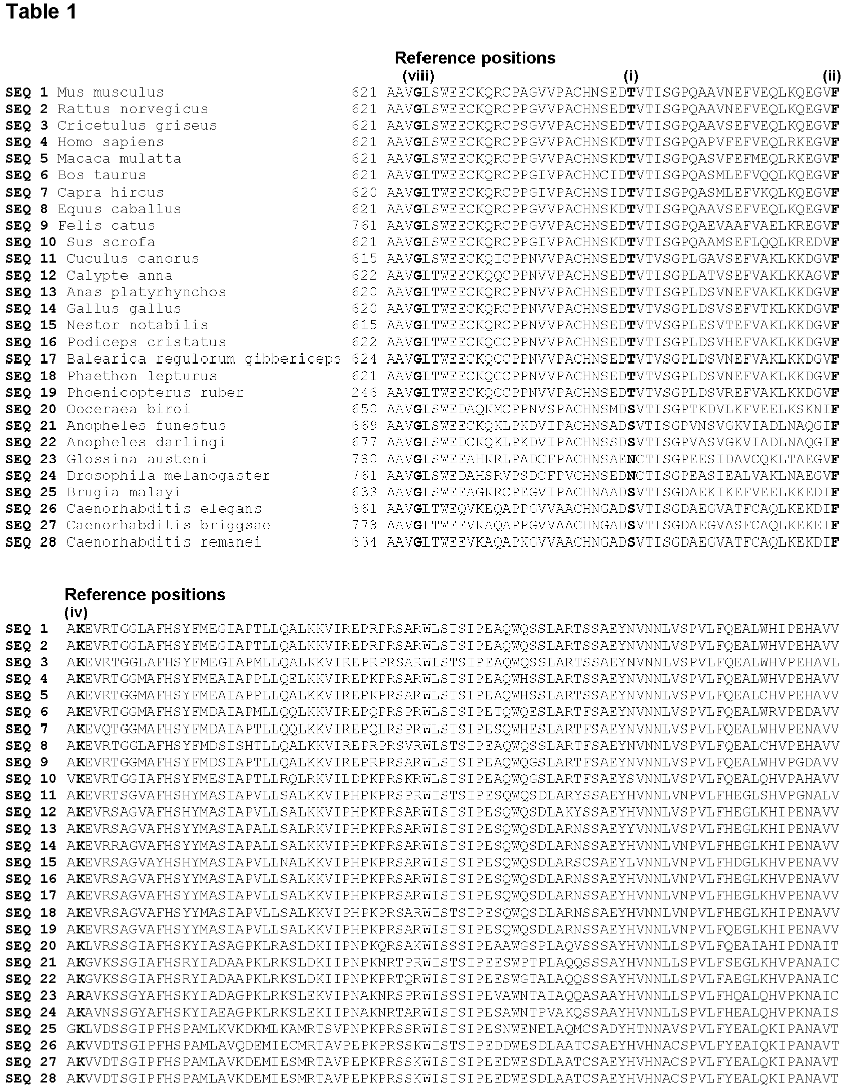

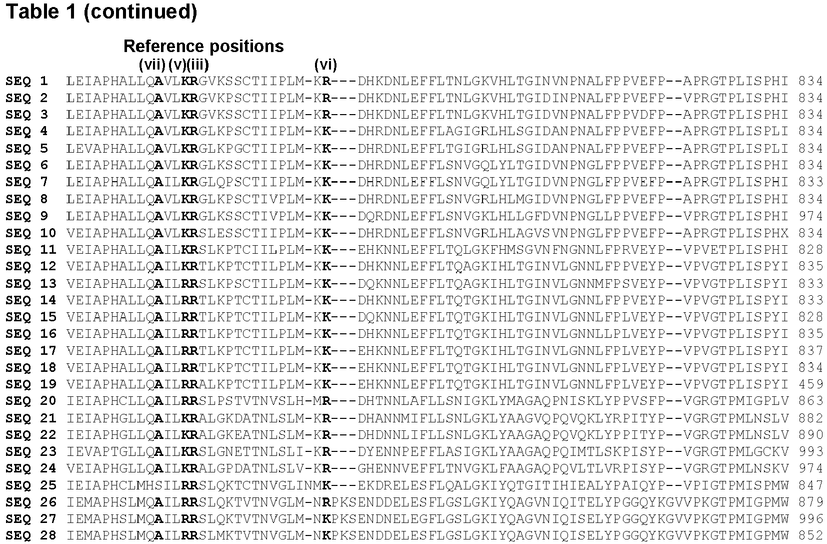

### Example 2: Cloning and expression of engineered acyltransferases

The basic plasmid encoding engineered murine acyltransferases was prepared from a synthetic DNA fragment, containing a KS-LD-MAT fragment including Strep- and His-tags and was cloned into a pET22b vector using the restriction enzymes Ndel and Xhol. Mutations in the eight different positions corresponding to the reference positions according to the present invention were introduced by PCR using primers harboring the nucleotide variations. The plasmid encoding for the ACP was prepared similarly. An additional ribosome binding site (RBS) and another gene encoding for the 4'-phosphopantetheinyl transferase Sfp was integrated into the same plasmid. The synthetic gene for the ACP was ordered with its native DNA sequence and codon-optimized for *E*. *coli.*

The PCR mixtures were treated with Dpn1 prior to DNA purification using electrophoresis. DNA was stained and illuminated with standard materials. Appropriate fragments were isolated with a scalpel and DNA was purified with standard commercial DNA purification kits. The fragments were transferred to Stellar^{™} Competent Cells (takahara) chemical competent cells for ligation and amplification of the targeted plasmids. The plasmids were isolated from cells using a standard commercial plasmid purification kit.

The plasmids encoding the MAT or engineered acyltransferases were transformed into BL21 (DE3) chemical competent cells. Transformed cells were directly transferred to a pre-culture of 5 mL LB-medium containing ampicillin as a selection marker, which was grown over night at 37°C in an incubation shaker. The pre-culture was used to inoculate a 100-250 mL main-culture in 2xYT or TB medium in a 500 mL Erlenmeyer flask. The culture was grown for 2 hours at 37°C and then the expression system was induced by adding 250 µM IPTG and was grown for another 16 hours at 20°C. The cells were lysed enzymatically in lysis buffer. After centrifugation, the soluble fraction was purified by Ni-NTA and Strep affinity chromatography following the standard protocol. Purified proteins were frozen in aliquots in liquid nitrogen and stored at -25°C. Protein concentrations were determined by their absorbance at 280 nm. The absorbance was recorded on a NanoDrop One^{C} (ThermoFisher) and converted to concentration using the extinction coefficient of the respective protein, calculated from their primary sequence without the N-terminal methionine in Benchling.

### Example 3: Kinetic analysis of engineered acyltransferases using MMal-CoA

An enzyme-coupled assay was performed to analyze the transfer of acyl-moieties from CoA to the ACP. Four solutions were prepared as 4x concentrated stocks in the assay buffer. Solution 1 contained the acyltransferase at the concentration range of 0.01 µM - 10 µM, depending on the transfer kinetics to achieve good signal to noise ratios. Solution 2 contained the enzyme-coupled system including 8 mM α-ketoglutaric acid, 1.6 mM NAD+, 1.6 mM TPP and 60 mU/100 µL αKGDH. Solution 3 contained the methylmalonyl (MMal)-CoA at concentrations between 0.1 µM and 1,000 µM and solution 4 contained the ACP from murine FAS as a standalone protein at 240 µM. 5 µL of Solutions 1-3 were pipetted into a 384-well Small Volume HiBase Microplates (Greiner Bio-one) and mixed manually. The reactions were started by adding solution 4 with the dispenser system of the microplate reader (ClarioStar, BMG labtech). The reaction progress was recorded using the following settings: 348-20 nm; emission: 476-20 nm; gain: 1500; focal height: 11.9 mm; flashes: 17; orbital averaging: off.

Kinetic data for exemplary mutants is shown in Table 2. Further analysis and verification is ongoing, including kinetic analysis of the mutants A282V, A282L, A137V, A137L, T161V, T161L, F184L/R286L, T161L/F184L/R286L, T161A/F184A/R286A/K186D/R300D, T161A/F184A/R286A/K186D/R300E, T161A/F184A/R286A/K186E/R300D, and T161A/F184A/R286A/K186E/R300E, corresponding to acyltransferases of SEQ ID NOs: 71 to 82.

**Table 2**

| **SEQ ID** | **Mutation** | ***K*ₘ [µM]** | ***k*_{cat} [s⁻¹]** | ***k*_{cat}*K*ₘ [M⁻¹ s⁻¹]** |
|---|---|---|---|---|
| 29* | none | 0.62 | 4.000 | 6.5E+06 |
| 29 | none | 0.53 | 1.297 | 2.5E+06 |
| 57 | T161A | 1.47 | 0.051 | 3.5E+04 |
| 58 | T161Y | 38.78 | 0.428 | 1.1E+04 |
| 59 | T161A F184A R286A | 3.41 | 0.021 | 6.2E+03 |
| 60 | T161A F184A K186A R286A | 7.24 | 0.013 | 1.8E+03 |
| 61 | K186E | 14.16 | 0.101 | 7.2E+03 |
| - | A282W | protein not stable | | |
| 62 | K186D | 18.63 | 0.093 | 5.0E+03 |
| 63 | K285D | 0.11 | 0.133 | 1.2E+06 |
| 65 | R300D | 5.06 | 0.582 | 1.2E+05 |
| 66 | R300E | 3.33 | 0.834 | 2.5E+05 |
| 67 | K186D R300D | 66.23 | 0.019 | 2.9E+02 |
| 68 | K186D R300E | 685.98 | 0.190 | 2.8E+02 |
| 69 | K186E R300D | 218.27 | 0.051 | 2.3E+02 |
| 70 | K186E R300E | 58.39 | 0.030 | 5.2E+02 |

Listed SEQ ID NOs refer to corresponding acyltransferase sequences.

Mutations are indicated in reference to the wild type MAT sequence SEQ ID NO: 29. *Comparative data from Rittner *et al.* 2018

### References

Hertweck C. The biosynthetic logic of polyketide diversity. Angew Chem Int Ed Engl. 2009;48(26):4688-716. doi: 10.1002/anie.200806121.
Rittner A, Paithankar KS, Huu KV, Grininger M. Characterization of the Polyspecific Transferase of Murine Type I Fatty Acid Synthase (FAS) and Implications for Polyketide Synthase (PKS) Engineering. ACS Chem Biol. 2018 Mar 16;13(3):723-732. doi: 10.1021 /acschembio.7b00718.
Rittner A, Paithankar KS, Himmler A, Grininger M. Type I fatty acid synthase trapped in the octanoyl-bound state. Protein Sci. 2020 Feb;29(2):589-605. doi: 10.1002/pro.3797.
Smith S, Tsai SC. The type I fatty acid and polyketide synthases: a tale of two megasynthases. Nat Prod Rep. 2007 Oct;24(5):1041-72. doi: 10.1039/b603600g.

## Claims

1. An acyltransferase having altered substrate specificity, wherein the acyltransferase comprises one or more amino acid exchange(s) at reference position(s)
(i) T161 according to SEQ ID NO: 29, or an analogous threonine, serine or asparagine residue at said reference position as defined in Table 1, wherein the amino acid is exchanged to alanine, glycine, valine, leucine, isoleucine, tyrosine, phenylalanine or tryptophan; and/or
(ii) F184 according to SEQ ID NO: 29, or an analogous phenylalanine residue at said reference position as defined in Table 1, wherein the amino acid is exchanged to alanine, glycine, valine, leucine, isoleucine or methionine; and/or
(iii) R286 according to SEQ ID NO: 29, or an analogous arginine residue at said reference position as defined in Table 1, wherein the amino acid is exchanged to alanine, glycine, valine, leucine, isoleucine or methionine; and/or
(iv) K186 according to SEQ ID NO: 29, or an analogous lysine or arginine residue at said reference position as defined in Table 1, wherein the amino acid is exchanged to a negatively charged amino acid or to glycine, alanine or valine; and/or
(v) K285 according to SEQ ID NO: 29, or an analogous lysine or arginine residue at said reference position as defined in Table 1, wherein the amino acid is exchanged to a negatively charged amino acid; and/or
(vi) R300 according to SEQ ID NO: 29, or an analogous lysine or arginine residue at said reference position as defined in Table 1, wherein the amino acid is exchanged to a negatively charged amino acid, and/or
(vii) A282 according to SEQ ID NO: 29, or an analogous alanine residue at said reference position as defined in Table 1, wherein the amino acid is exchanged to valine, leucine, isoleucine, phenylalanine or tyrosine; and/or
(viii) G137 according to SEQ ID NO: 29, or an analogous glycine residue at said reference position as defined in Table 1, wherein the amino acid is exchanged to valine, leucine, isoleucine, phenylalanine or tyrosine.

2. The acyltransferase of claim 1, wherein
an amino acid exchange at reference position (i) is an exchange to alanine, glycine, or valine, preferably to alanine; and/or wherein
an amino acid exchange at reference position (ii) is an exchange to alanine, glycine, or valine, preferably to alanine; and/or wherein
an amino acid exchange at reference position (iii) is an exchange to alanine, glycine, or valine, preferably to alanine; and/or wherein
an amino acid exchange at reference position (iv) is an exchange to a negatively charged amino acid, preferably to glutamate or aspartate; and/or wherein
an amino acid exchange at reference position (v) is an exchange to glutamate or aspartate; and/or wherein
an amino acid exchange at reference position (vi) is an exchange to glutamate or aspartate; and/or wherein
an amino acid exchange at reference position (vii) is an exchange to leucine, isoleucine, phenylalanine or tyrosine; and/or wherein
an amino acid exchange at reference position (viii) is an exchange to leucine, isoleucine, phenylalanine or tyrosine.

3. The acyltransferase of claim 1, wherein
an amino acid exchange at reference position (i) is an exchange to leucine, isoleucine, tyrosine, phenylalanine or tryptophan; and/or wherein
an amino acid exchange at reference position (ii) is an exchange to leucine, isoleucine or methionine; and/or wherein
an amino acid exchange at reference position (iii) is an exchange leucine, isoleucine or methionine; and/or wherein
an amino acid exchange at reference position (iv) is an exchange to alanine, glycine, or valine; and/or wherein
an amino acid exchange at reference position (vii) is an exchange to valine; and/or wherein
an amino acid exchange at reference position (viii) is an exchange to valine.

4. The acyltransferase of any of the preceding claims, wherein the acyltransferase is an isolated acyltransferase originating from a fatty acid synthase.

5. The acyltransferase of claim 1, wherein the acyltransferase comprises amino acid exchanges as defined in claim 1 at reference positions (iv) and (i), or (iv) and (ii), or (iv) and (iii), or (iv) and (v), or (iv) and (vi), or (iv) and (vii), or (iv) and (viii), or (iv) and two or more of reference positions of (i), (ii), (iii), (v), (vi), (vii) and/or (viii), optionally wherein the amino acid exchange at reference position (iv) is an amino acid exchange to glutamate or aspartate.

6. The acyltransferase of claim 1, wherein the acyltransferase comprises amino acid exchanges as defined in claim 1 at reference positions (i) and (ii), or (i) and (iii), or (i) and (iv), or (i) and (v), or (i) and (vi), or (i) and (vii), or (i) and (viii), or (i) and two or more of reference positions of (ii), (iii), (iv), (v), (vi), (vii) and/or (viii), optionally wherein the amino acid exchange at reference position (i) is an amino acid exchange to alanine.

7. The acyltransferase of claim 1, wherein the acyltransferase comprises amino acid exchanges as defined in claim 1 at reference positions:
(i) and (ii) and (iii) and optionally (iv), preferably wherein the amino acids at said reference positions are exchanged to alanine; or
(ii) and (iii) and optionally (i), preferably wherein the amino acids at said reference positions are exchanged to alanine; or
(iv) and (vi), preferably wherein the amino acid exchanges at said reference positions are amino acid exchanges to glutamate at reference positions (iv) and (vi), or amino acid exchanges to aspartate at reference positions (iv) and (vi), or an amino acid exchange to glutamate at reference position (iv) and an amino acid exchange to aspartate at reference position (vi), or an amino acid exchange to aspartate at reference position (iv) and an amino acid exchange to glutamate at reference position (vi), optionally further comprising amino acid exchanges as defined in claim 1 at reference positions (i) and (ii) and (iii), preferably amino acid exchanges to alanine at reference positions (i) and (ii) and (iii).

8. The acyltransferase of any of the preceding claims, wherein the acyltransferase originates from a fatty acid synthase of an organism selected from vertebrata, including aves and mammalia, including artiodactyla, perissodactyla, carnivora, primates and rodentia, optionally wherein the acyltransferase originates from a fatty acid synthase comprising or consisting of a wild type amino acid sequence selected from any one of SEQ ID NO: 1 to 28, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

9. The acyltransferase of any of the preceding claims, wherein the acyltransferase comprises or consists of an amino acid sequence of any of SEQ ID NO: 57 to 82, or a sequence having at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or at least 99 % sequence identity thereto.

10. A fusion protein comprising at least one acyltransferase of any one of the preceding claims, preferably wherein the fusion protein further comprises at least one linker domain and optionally at least one ketoacyl synthase domain.

11. A nucleic acid encoding the acyltransferase of any one of claims 1 to 9, or the fusion protein of claim 10.

12. An expression construct or vector comprising at least one nucleic acid of claim 11.

13. A cell comprising at least one acyltransferase of any one of claims 1 to 9; and/or at least one fusion protein of claim 10; and/or at least one nucleic acid of claim 11; and/or at least one expression construct or vector of claim 12.

14. A kit comprising at least one acyltransferase of any one of claims 1 to 9; and/or at least one fusion protein of claim 10; and/or at least one nucleic acid of claim 11; and/or at least one expression construct or vector of claim 12; and/or at least one cell of claim 13; optionally further comprising at least one container; and/or a set of reagents including buffer, medium and/or substrate(s); and/or means of transfection.

15. A use of at least one acyltransferase as defined in any one of claims 1 to 9; and/or at least one fusion protein as defined in claim 10; and/or at least one nucleic acid as defined in claim 11; and/or at least one expression construct or vector as defined in claim 12; and/or at least one cell as defined in claim 13; and/or at least one kit as defined in claim 14; for polyketide synthesis, fatty acid synthesis, and/or nonribosomal peptide synthesis, optionally wherein it is a use for reacting at least two different substrates.
